# EUROPEAN PATENT SPECIFICATION

(11) **EP 1 911 358 B1**
(45) Date of publication and mention of the grant of the patent: **17.06.2009**
(21) Application number: 07118425.3
(22) Date of filing: 12.10.2007
(51) Int. Cl.: A23L 1/30, A23L 1/305, A61K 8/368, A61K 8/97, A61K 8/99, A61K 31/202, A61K 31/353, A61K 36/82, A23C 9/152, A61P 17/00

(54) **New composition for improving skin quality and a process for preparing the same**
Neue Zusammensetzung zur Verbesserung des Hautzustandes und Verfahren zu ihrer Herstellung
Nouvelle composition pour améliorer la qualité de la peau et son procédé de préparation

(30) Priority: 13.10.2006 EP 06291607
(43) Date of publication of application: 16.04.2008
(73) Proprietor: COMPAGNIE GERVAIS DANONE, 75009 Paris (FR)
(72) Inventor: Guyonnet, Denis, 92300 Levallois Perret (FR); Lassel, Taous, 75006 Paris (FR); Samson-Villeger, Sandrine, 92160 Antony (FR); Trgo, Christian, 76230 Bois Guillaume (FR); Rawlings, Anthony Vincent, Kingsmead Northwich, Cheshire CW9 8FH (GB)
(74) Representative: Grosset-Fournier, Chantal Catherine

(56) References cited:
- WO-A-20/06015675
- WO-A-20/06056293
- WO-A-20/07015027
- FR-A1- 2 861 594
- JP-A- 2003 063 942
- JP-A- 2006 246 861
- US-A1- 2004 258 645

## Description

The present invention relates to a new composition for improving skin quality, in particular by acting on skin barrier function or skin moisturizing barrier, and a process for preparing the same.

The main functions of the skin are to provide mechanical protection (from friction and loading), to provide a chemical barrier (limitation of the foreign substance penetration, prevention of water or endogenous fluid loss, constant temperature keeping), to protect against ultraviolet irradiation, and to protect against pathogen invasion (thanks immune cells located in the skin).

The primary layer that provides this protection is the stratum corneum (SC). This layer prevents excessive water loss from the body because too much water loss is a sign of skin dehydration.

The cosmetic and dermatological industry aims to prevent the skin dehydration, the appearance of dry skin, by the topical application of creams and lotions containing ingredients that can improve the skin condition. These effects are, however, only local effects i.e. the benefit is only provided to the part of the skin where the lotion is applied.

However, the body provides the skin with a unique delivery system, i.e. the blood and interstitial fluids, and as a result any nutrients (or their metabolites) that are imbibed orally have the opportunity via this delivery route to influence the skin of the whole of the body.

Examples for this are disclosed in e.g., JP 2006246861; FR 2861594; US 2004/0258645; and WO 2006/056293.

So the use of ingredients able to provide protection to the skin, particularly in orally administrated compositions, is beneficial to improve skin condition of the whole of the body.

The importance of polyunsaturated fatty acids for skin barrier function has been known for decades. Gamma-linolenic acid (GLA) may act at several different levels to improve skin barrier function:
- by possessing an anti-inflammatory activity, GLA may reduce the keratinocyte hyperproliferation that occurs in barrier compromised conditions to allow normal keratinocyte differentiation to occur;
- as a ligand for the peroxisomal proliferator activated receptor (PPAR), it may influence keratinocyte gene transcription and induce its differentiation directly leading to increased ceramide synthesis.
   Polyphenols such as catechins have been shown to improve stratum corneum (SC) barrier function (Heinrich U et al., Long term ingestion of high flavanol cocoa provides photoprotection against UV-induced erythema and improves skin condition in women. J Nutr 136: 1565-9, 2006). Green tea polyphenols have many effects on skin cells either directly or possibly by their conversion into small aromatic acids which could then function as PPAR ligands. Catechins have known effects on keratinocyte differentiation (Balasubramanian S et al., GTP stimulates a Ras, MEKK1, MEK3 & p38 cascade to increase activator protein 1 factor dependent involucrin gene expression in normal human keratinocytes. JBC. 2002; 277: 1828-1836; Eckert RL, Crish JF, Efimova T, Balasubramanian S. Opposing action of curcumin and green tea polyphenol in human keratinocytes. Mol Nutr Food Res. 2006 Feb;50(2): 123-9; Hsu S, Bollag WB, Lewis J et al., Green tea polyphenols induce differentiation and proliferation in epidermal keratinocytes. J Pharmacol Exp Ther. 2003 Jul;306(1):29-34, 2003).

The present invention results from the fact that it has unexpectedly been found that polyunsaturated fatty acids, which are well known in the art for their action on the skin, present an improved bioavailability in certain conditions.

One of the aspects of the invention is to propose new compositions useful as a food, a beverage or a supplement composition for a food or a beverage which is intended for the improvement of the skin hydration and the prevention of the skin dehydration.

Another aim of the invention is to provide a new composition having a very good organoleptic quality (not astringent, not too thick, not too bitter) from the consumer point of view and a good nutritional balance (i.e low fat content < 3.5% w/w), said composition being also not too expensive.

Another aspect of the invention is to propose a process for preparing the compositions.

The present invention relates to a composition comprising, as active substance, a mixture of at least one polyunsaturated fatty acid, different from polyunsaturated fatty acid derived from milk, polyphenols, milk proteins and lactic bacteria, and said composition having a water content of at least 50% by weight; and wherein the said polyunsaturated fatty acid presents an increased bioavailability compared with the one of the polyunsaturated fatty acid alone.

As it is well-understood in the art, the term "polyunsaturated fatty acid" refers to fatty acids having from 1 to 26 carbon atoms, particularly from 18 to 24 carbon atoms and more particularly 18 carbon atoms and from 2 to 6, particularly 2 to 4 and more particularly 3 unsaturations.

The term "polyunsaturated fatty acid" as used herein, designates polyunsaturated fatty acids which are different from polyunsaturated fatty acids which are usually found in milk.

The expression "polyunsaturated fatty acid, different from polyunsaturated fatty acid derived from milk" designates polyunsaturated fatty acids which may have a chemical structure different from the one of the polyunsaturated fatty acids found in milk, or the amount of which is different from the one found in milk.

It should also be understood that the composition of the invention, comprising an active substance as defined above, can comprise polyunsaturated fatty acids which are of dairy substance origin, said dairy substance being defined hereafter.

Examples of polyunsaturated fatty acids derived from milk are linoleic acid (C18:2 ω-6), alpha-linolenic acid (C18:3 ω-3) or conjugated linoleic acids (CLA). The milk does not contain gamma-linolenic acid (GLA).

The term "lactic bacteria" designates the family of Gram-positive bacteria, in shape of hulls or sticks capable to ferment sugars in lactic acid.

The term "bioavailability" designates the capacity of an ingredient to be available in an active form in the blood, the said ingredient being available for metabolic process or storage in the skin.

The term "increased bioavailability" designates the capacity to improve ingredient bioavailability. It could be measured by taking a blood sample and make ingredient dosage at different times.

The term "polyunsaturated fatty acid alone" designates polyunsaturated fatty acids which are either present in their natural environment, or isolated from said environment, and without being in combination with elements liable to affect their properties.

It has unexpectedly been found that polyunsaturated fatty acids in combination with polyphenols, milk proteins and lactic bacteria present a better kinetic of bioavailibility compared with polyunsaturated fatty acids alone.

The present invention is defined by the claims.

The present invention also relates to a composition comprising, as active substance, a mixture of at least one gamma-linolenic acid, polyphenols, milk proteins and lactic bacteria, and said composition having a water content of at least 50% by weight, and wherein the said gamma-linolenic acid presents an increased bioavailability compared with the one of the gamma-linolenic acid alone.

The term "the said gamma-linolenic acid presents an increased bioavailability compared with the one of the gamma-linolenic acid alone" designates the capacity of gamma-linolenic acid of the composition to be more available in an active form in the blood than the gamma-linolenic acid alone. The gamma-linolenic acid in combination with polyphenols, milk proteins and lactic bacteria present a better kinetic of bioavailibility compared with gamma-linolenic acid alone.

The present invention also relates to a composition comprising, as active substance, a mixture of at least one gamma-linolenic acid, polyphenols, milk proteins and lactic bacteria, and said composition having a water content of at least 50% by weight, and wherein the concentration of gamma-linolenic acid is from about 0.02% to about 1%, particularly from about 0.04% to about 0.6% and more particularly from about 0.1% to about 0.2% by weight of the composition, and wherein the said gamma-linolenic acid presents an increased bioavailability compared with the one of the gamma-linolenic acid alone.

The present invention also relates to a composition comprising, as active substance, a mixture of at least one gamma-linolenic acid, polyphenols, milk proteins and lactic bacteria, and said composition having a water content of at least 50% by weight, and wherein the concentration of gamma-linolenic acid is from about 0.02% to about 1%, particularly from about 0.04% to about 0.6% and more particularly from about 0.1% to about 0.2% by weight of the composition, and the concentration of polyphenols represent about 0.13% to 18.7%, particularly about 0.15% to 15%, and more particularly about 0.16% to 13% of the total weight of the active substance, and wherein the said gamma-linolenic acid presents an increased bioavailability compared with the one of the gamma-linolenic acid alone.

The present invention relates to a composition comprising, as active substance, a mixture of at least one polyunsaturated fatty acid, different from polyunsaturated fatty acid derived from milk, polyphenols, milk proteins and lactic bacteria, and said composition having a water content of at least 50% by weight, and wherein the concentration of polyphenols represent about 0.13% to 18.7%, particularly about 0.15% to 15%, and more particularly about 0.16% to 13% of the total weight of the active substance, and wherein the said polyunsaturated fatty acid presents an increased bioavailability compared with the one of the polyunsaturated fatty acid alone.

In an advantageous embodiment, in the composition of the present invention, the polyunsaturated fatty acid is contained in one at least of the following vegetable oils: borage oil, primrose oil, black currant oil, camelina oil, hemp seed oil, kiwi seed oil, flax oil, rapeseed oil, and micro algae oil or is produced by a enzymatic process.

The expression "enzymatic process" as used herein designates the catalytic action of specific proteins which could chemically transform some fatty acids.

In another advantageous embodiment, in the active substance of the composition of the present invention, the polyunsaturated fatty acid is selected among linolenic acids, their derivatives and their precursors, and is in particular gamma-linolenic acid.

The term "precursors" designates molecules which can provide gamma-linolenic acid, following a specific treatment as enzymatic, chemical or physical treatment.

The term "derivatives" designates molecules which can be produced by using gamma-linolenic acid through an enzymatic, chemical or physical treatment.

In an advantageous embodiment, in the composition of the present invention, polyphenols are contained in a vegetal extract such as green tea extract, grape seed extract, grape skin extract, grapefruit, (red) orange extract, pine bark extract (pycnogenol), red wine extract, tomato extract, gingko biloba extract, cranberry extract, soy extract, soy isoflavons extract, blueberry, raspberry, elderberry, pomegranate, apple, cider, cocoa, green coffee, mango, cactus pear, black currant, seaweeds, olive, cinnamon, ginger, ginseng, aloe vera, brocoli, hibiscus.

More preferably, the vegetal extract comprises at least 70%, particularly at least 80% of polyphenols by weight of the vegetal extract.

In another advantageous embodiment, in the active substance of the composition of the present invention, polyphenols are selected among: phenolic acids, such as hydroxycinnamic acids, hydroxybenzoic acids; flavonoids such as flavonols, flavones, isoflavones, flavanones, anthocyanidins, proanthocyanidins; hydrolyzable tannins such as gallotannins, ellagitannins and are in particular tea catechins, resveratrol, viniferins, caffeic acid, gallic acid, ellagic acid, ferulic acid, chlorogenic acid, quercetin, kaempferol, apigenin, daidzein, genistein, naringenin, hesperidin, anthocyanin, catechin, epicatechin, and gallocatechin.

In another advantageous embodiment, in the active substance of the composition, polyphenols are selected among tea catechins.

More preferably, polyphenols comprise at least 70%, particularly at least 80% of catechins by weight of the total polyphenol content of the vegetal extract.

In another advantageous embodiment, in the active substance of the composition at least 50%, more particularly at least 60% by weight of tea catechins are selected among epicatechin, epigallocatechin, and epigallocatechin gallate.

In an advantageous embodiment, in the composition of the present invention, milk proteins are contained in one at least of the following dairy substance: milk, skimmed milk, milk powder, skimmed milk powder, cream, milk protein concentrate, whey, whey protein concentrate, and caseinate.

The expression "dairy substance" is utilized to refer to the initial milk-based composition which has undergone a treatment in particular a heat treatment. The expression "initial milk-based composition" designates the starting dairy composition before any treatment.

The dairy substance generally contains polyunsaturated fatty acids, which are not in a sufficient amount to confer the properties to the composition. That is why it is necessary to add additional polyunsaturated fatty acids to increase the total amount of them, at least one of them being different from those contained in the dairy substance.

The expression "milk protein concentrate" designates milk protein prepared *via* milk ultrafiltration or other means such that the lactose or salt content is reduced, thereby enhancing the protein content. This concentrate could contain from 42 to 85% of proteins.

The expression "whey protein concentrate" designates whey protein prepared *via* the process cited above. This concentrate could contain from 35 to 89% of proteins.

Some protein contents of other dairy substances are given below, in table 1.

**Table 1: Proteins concentrations of other dairy substances**

| Dairy substances | Protein concentrations |
|---|---|
| Whole milk | 3.2 % |
| Skimmed milk | 3.3-3.6% |
| Whole milk powder (27% fat content) | 27% |
| Skimmed milk powder | 35-37% |
| Cream (40% of fat) | 1.85%-2.1% |
| Caseinate | > 90% |

In another advantageous embodiment, in the active substance of the composition of the present invention, lactic bacteria are chosen among one at least of the following bacteria: *Streptococcus thermophilus, Lactobacillus delbrueckii ssp. bulgaricus, Lactobacillus casei, Lactobacillus paracasei, Lactobacillus acidophilus, Lactobacillus helveticus, Lactobacillus plantarum, Lactobacillus reuteri, Lactobacillus kefiranofaciens, Bifidobacterium lactis ssp animalis, Bifidobacterium longum, Bifidobacterium breve, Lactococcus lactis ssp cremoris, Lactococcus lactis ssp lactis* and *Leuconostoc.*

In a particularly advantageous embodiment, in the active substance of the composition of the present invention, lactic bacteria are *Streptococcus thermophilus, Lactobacillus bulgaricus,* and *Lactobacillus casei.*

More preferably, *Lactobacillus casei* is the strain CNCM-1518.

Other examples of strains which could be used are the following: I-2116, ATCC 53103, CRL 431, and I-1225.

In another advantageous embodiment, in the composition of the present invention, the water content is from 70% to 90 %, particularly 75% to 85 %, more particularly 80% by weight of the composition.

In an advantageous embodiment, in the active substance of the composition of the present invention,
- polyunsaturated fatty acids represent about 2.3% to 91.5%, particularly about 2.3% to 70% and more particularly about 2.3% to 65% of the total weight of the active substance;
- polyphenols represent about 0.13% to 18.7%, particularly about 0.15% to 15%, and more particularly about 0.16% to 13% of the total weight of the active substance;
- milk proteins represent about 8% to 97.6%, particularly 15% to 97.6% and more particularly about 30% to 97.4% of the total weight of the active substance;
- lactic bacteria are present in the active substance in an amount of about 10⁶ to 10¹⁰ cfu/mg, particularly 10⁷ to 10⁹ cfu/mg.

In an advantageous embodiment, in the active substance of the composition of the present invention,
- the polyunsaturated fatty acids comprise gamma-linolenic acid, which represents about 0.64% to 28%, particularly about 1% to 10% and more particularly about 2% to 4% of the total weight of the active substance;
- the polyphenols comprise tea catechins, which represent about 0.15% to 11.5%, particularly about 0.5% to 5%, and more particularly about 1% to 2% of the total weight of the active substance;
- milk proteins represent about 65% to 99%, particularly 80% to 97% and more particularly about 90% to 95% of the total weight of the active substance;
- lactic bacteria are present in the active substance in an amount of about 10⁶ to 10¹⁰ cfu/mg, particularly 10⁷ to 10⁹ cfu/mg.

In the case of the use of oils with a low GLA concentration (e.g hemp seed oil containing 76.5% of PUFA including 2.7% of GLA) and low concentrated vegetal extracts (e.g green tea extract containing 90% of polyphenols including 30% of catechins), the active substance of the composition comprises:
- a concentration of polyunsaturated fatty acids from about 14 to about 91.5% of the total weight of the active substance;
- a concentration of polyphenols from about 0.13% to about 18.7% of the total weight of the active substance;
- a concentration of milk proteins from about 8% to about 84% of the total weight of the active substance; and
- an amount of lactic bacteria of about 10⁶ to 10¹⁰ cfu/mg, particularly 10⁷ to 10⁹ cfu/mg.

In the case of the use of oils with a high GLA concentration (e.g borrage oil, evening primrose oil including 10% of GLA, black currant oil including 17% of GLA) and very concentrated vegetal extracts (e.g green tea extract containing 90% of polyphenols including 80% of catechins), the active substance of the composition comprises:
- a concentration of polyunsaturated fatty acids from about 2.3% to about 60% of the total weight of the active substance;
- a concentration of polyphenols from about 0.16% to about 12.3% of the total weight of the active substance;
- a concentration of milk proteins from about 37.6% to about 97.4% of the total weight of the active substance; and
- an amount of lactic bacteria of about 10⁶ to 10¹⁰ cfu/mg, particularly 10⁷ to 10⁹ cfu/mg.

In another advantageous embodiment, in the composition of the present invention, the concentration of polyunsaturated fatty acids is from about 0.15% to about 16.6%, particularly from about 0,2% to about 10% and more particularly from about 0.4% to about 5% by weight of the composition.

In another advantageous embodiment, in the composition of the present invention, the concentration of gamma-linolenic acid is from about 0.02% to about 1%, particularly from about 0.04% to about 0.6 % and more particularly from about 0.1 % to about 0.2% by weight of the composition.

If the concentration of gamma-linolenic acid is lower than 0.02% by weight of the composition, no significant effect is observed and, if the concentration of gamma-linolenic acid is grater than 1% by weight of the composition, the product does not have the required organoleptic quality.

In another advantageous embodiment, in the composition of the present invention, the gamma-linolenic acid / tea catechins ratio is of about 2 to about 10, particularly about 3 to about 5 and more particularly about 3.4.

If the gamma-linolenic acid / tea catechins ratio is lower than 2, no significant effect is observed, and if the gamma-linolenic acid / tea catechins ratio is greater than 10, the product does not have the required organoleptic quality.

In another advantageous embodiment, in the composition of the present invention, the concentration of milk proteins is from about 1.5% to about 6%, particularly from about 2.5% to about 5% by weight of the composition.

If the concentration of milk proteins is lower than 1.5% by weight of the composition, the composition has a bad nutritional profile with a bad organoleptic quality (very watery), and if the concentration of milk proteins is greater than 6% by weight of the composition, the composition is very thick and too astringent.

In another advantageous embodiment, in the composition of the present invention, the amount of lactic bacteria is more than 10⁶ up to 10¹⁰ cfu/mg, particularly 10⁷ to 10⁹ cfu/mg.

The amount of lactic bacteria can be measured 28 days after conditioning and storage at a temperature from 4 to 10°C.

If the amount of lactic bacteria is lower than 10⁶ cfu/mg, the composition has not the optimal required effect, and if the amount of lactic bacteria is greater than 10¹⁰ cfu/mg, the composition is technically difficult to manufacture and then is too expensive.

In another advantageous embodiment, in the composition of the present invention, the concentration of the vegetable oil, in particular borage oil, is from about 0.2 to about 3% by weight of the composition.

If the concentration of the vegetable oil is lower than 0.2% by weight of the composition, the composition will not have the optimal required effect because it will not contain enough active ingredient, and if the concentration of the vegetable oil is greater than 3% by weight of the composition, the composition will have a too high fat content, will have some sensorial issues and will be very expensive.

In another advantageous embodiment, in the composition of the present invention, the concentration of the vegetal extract, in particular green tea extract, is from about 0.01 % to about 1% by weight of the composition.

If the concentration of the vegetable extract is lower than 0.01% by weight of the composition, the composition will not have the optimal required effect, and if the concentration of the vegetable extract is grater than 1% by weight of the composition, the composition will have some sensorial issues and will be very expensive.

In a typical embodiment of the present invention, the composition comprises borage oil, green tea extract, milk, and lactic bacteria, and has a water content of at least 50% by weight, and wherein the polyunsaturated fatty acid contained in borage oil presents an increased bioavailability compared with the one of the polyunsaturated fatty acid alone.

In another advantageous embodiment, in the composition of the present invention, the borage oil comprises from about 16% to about 25%, and particularly about 20% of gamma-linolenic acid by weight of the borage oil.

The borage oil is the selected source of Gamma-Linolenic Acid or GLA as it possesses the highest GLA content of any available oil seed. It is also a significant source of the essential linoleic acid (C18:2 ω-6). Results of its composition during analytical quality control testing have shown that the borage oil used satisfies not only the product specification but also the regulatory requirements regarding microbiological and toxicological contaminants such as pesticides, polycyclic aromatic compounds, and heavy metals.

In borage oil, GLA is concentrated in the sn-2 position in the triglycerides and is more bioavailable than in evening primrose oil in which GLA is concentrated in the sn-1 and sn-3 positions in the triglycerides. The triacylglycerol stereospecific structure of these oils is distinct. This difference of position influence GLA bioavailability.

In another advantageous embodiment, in the composition of the present invention, the green tea extract comprises from about 50% to about 90%, and particularly about 80% of catechin by weight of the green tea extract.

The green tea extract is made from high quality green tea leaves from the *Camellia sinensis* plant. The extract is mainly composed of polyphenols, especially catechins. The three following derivatives are the main components: epigallocatechin gallate (> 40%), epigallocatechin (> 18%), and epicatechin (>8%). Other minor catechins are also represented: catechin, gallocatechin, epicatechin gallate, and gallocatechin gallate. Furthermore, analytical measurements carried out regularly by accredited laboratories showed that the used green tea extract satisfies the microbiological and toxicological requirements.

In another advantageous embodiment, in the composition of the present invention, the dairy substance comprises from about 1.5% to about 6%, and particularly about 2.5% to about 5% of milk proteins by weight of the dairy substance.

In a typical embodiment of the present invention, the composition is a food or a beverage or a supplement composition for a food or a beverage.

In a particularly embodiment, the food is selected among a fermented dairy product containing at least 50% of the fermented product derived from the dairy substance, a yoghurt and a fruit yoghurt.

The expression "fermented dairy product" designates a product containing milk which has been fermented by a lactic bacteria. For example, the fermented dairy products which could be cited are the following: yoghurts such as set or stirred yoghurts, plain or flavoured yoghurts or yoghurts with fruits, fermented milks, fresh cheese, such as plain or flavoured fresh cheese or fresh cheese with fruits, and desserts.

The expression "fermented milk" and "yoghourts" could be understood as being notably in accordance with the official standards as Codex Alimentarius (notably vol. 12 and standard "Codex Stan 1-11 (a)-1975) or French decree n°88-1203 dated December 31^{st}, 1988.

In an advantageous embodiment of the present invention, the composition is intended to improve skin quality by acting on skin barrier function or skin moisturizing barrier.

It enables to improve the skin hydration. More particularly, the composition is intended for the decrease of skin water loss and for the improvement of skin quality.

Water loss is due to evaporation which is calculated in determining the pressure gradient of the water vapour layer surrounding the skin. More specifically, this technique enables the evaluation of the barrier effect of the stratum corneum and the hydrolipidic film.

TransEpidermal Water Loss (TEWL) measurements will be done with a SERVOMED EP2® "EVAPORIMETER." A flow of water vapour goes through a probe made up of two captors. The difference of the partial pressure is measured between the two captors. This value corresponds to the evaporation speed of a volatile substance (in this case, water). One measurement per zone will be carried out and the results of the TEWL measurements will be done in g/m²/h. This methodology is well known by the skilled person.

The present invention also relates to a process for preparing a composition comprising, as active substance, a mixture of at least one polyunsaturated fatty acid different from polyunsaturated fatty acid derived from milk, polyphenols, milk proteins and lactic bacteria and having a water content of at least 50% by weight; and wherein the concentration of polyphenols represents about 0.13 to 18.7% of the total weight of the active substance; and wherein the said polyunsaturated fatty acid presents an increased bioavailability compared with the one of the polyunsaturated fatty acid alone,
said process comprising the addition of a vegetal extract containing polyphenols to a fermented homogenized mixture of a vegetable oil containing polyunsaturated fatty acids or enzymatically derived polyunsaturated fatty acids and of a dairy substance containing milk proteins.

The expression "enzymatically derived polyunsaturated fatty acids" designates polyunsaturated fatty acids produced by a enzymatic process.

In an advantageous embodiment of the present invention, the process comprises the preparation of a fermented homogenized mixture of a vegetable oil containing polyunsaturated fatty acids or enzymatic ally derived polyunsaturated fatty acids and of a dairy substance containing milk proteins by:
- a step of incorporation of a vegetable oil or enzymatically derived polyunsaturated fatty acids within a dairy substance to obtain a mixture of a vegetable oil or enzymatically derived polyunsaturated fatty acids and of a dairy substance;
- a step of homogenisation of the above-mentioned mixture to obtain a homogenized mixture of a vegetable oil or enzymatically derived polyunsaturated fatty acids and of a dairy substance;
- a step of fermentation of the above-mentioned homogenized mixture to obtain a fermented homogenized mixture of a vegetable oil or enzymatically derived polyunsaturated fatty acids and of a dairy substance.

The present invention also relates to a composition such as obtained by the process described above.

The present invention also relates to a process for preparing a composition such as described above, said process comprising the addition of a vegetal extract containing polyphenols to a fermented homogenized mixture of a vegetable oil containing polyunsaturated fatty acids or of enzymatically derived polyunsaturated fatty acids and of a dairy substance containing milk proteins.

The present invention also relates to a process for preparing a composition such as described above, said process comprising the addition of a vegetal extract containing polyphenols to a fermented homogenized mixture of a vegetable oil containing polyunsaturated fatty acids or of enzymatically derived polyunsaturated fatty acids and of a dairy substance containing milk proteins, and said process comprising the preparation of a fermented homogenized mixture of a vegetable oil containing polyunsaturated fatty acids or enzymatically derived polyunsaturated fatty acids and of a dairy substance containing milk proteins by:
- a step of incorporation of a vegetable oil or enzymatically derived polyunsaturated fatty acids within a dairy substance to obtain a mixture of a vegetable oil or enzymatically derived polyunsaturated fatty acids and of a dairy substance;
- a step of homogenisation of the above-mentioned mixture to obtain a homogenized mixture of a vegetable oil or enzymatically derived polyunsaturated fatty acids and of a dairy substance;
- a step of fermentation of the above-mentioned homogenized mixture to obtain a fermented homogenized mixture of a vegetable oil or enzymatically derived polyunsaturated fatty acids and of a dairy substance.

In an advantageous embodiment, in the process of the present invention, the dairy substance is prepared by blending skimmed milk with milk powder or concentrated milk, followed-up by a treatment which is intended to eliminate bacteriological contaminants, particularly a heat treatment of the dairy substance, in particular by heating at 95°C during 4-8 min.

In another embodiment of the present invention, the process comprises:
- the preparation of a fermented homogenized mixture of a vegetable oil containing polyunsaturated fatty acids or of enzymatically derived polyunsaturated fatty acids and of a dairy substance containing milk proteins by:
   -- a step of incorporation on line of a vegetable oil or enzymatically derived polyunsaturated fatty acids within a dairy substance, said dairy substance being prepared by the blend of skimmed milk with milk powder or concentrated milk, followed-up by a treatment which is intended to eliminate bacteriological contaminants, particularly a heat treatment of the dairy substance, in particular by heating at 95°C during 4-8 min, to obtain a mixture of a vegetable oil and of a dairy substance;
   -- a step of homogenisation at 50-300 bars at 40-95°C of the above-mentioned mixture to obtain a homogenized mixture of a vegetable oil or enzymatically derived polyunsaturated fatty acids and of a dairy substance;
   -- a step of fermentation of the above-mentioned homogenized mixture, said fermentation comprising incorporation of ferments at 30-45°C during 5-10h, to obtain a fermented homogenized mixture of a vegetable oil or enzymatically derived polyunsaturated fatty acids and of a dairy substance;
- the addition via fruit preparation or sugar slurry or water based tea infusion of a vegetal extract containing polyphenols to the fermented homogenized mixture of a vegetable oil or enzymatically derived polyunsaturated fatty acids and of a dairy substance.

The present invention also relates to a composition such as described above, comprising an emulsion which contains polyunsaturated fatty acids different from polyunsaturated fatty acid derived from milk and milk proteins, in which 90% of fat particles of this emulsion have a maximum diameter of about 1.15 µm, 50% of fat particles of this emulsion have a maximum diameter of 0.5 µm and 10% of fat particles of this emulsion have a maximum diameter of 0.21 µm.

The term "emulsion" designates a mixture of two immiscible (unblendable) liquid substances. One substance (the dispersed phase) is dispersed in the other (the continuous phase). Emulsions are unstable and thus do not form spontaneously. Energy input through shaking, stirring, homogenizers, or spray processes is needed to form an emulsion. Over time, emulsions tend to revert to the stable state of liquid part separated from aqueous part. Surface active substances (surfactants) can increase the kinetic stability.

In the invention, the term "fat particles" designates fat dispersed in aqueous phase surrounded by milk proteins. Fat particles contain PUFA and all other fatty acids.

The present invention also concerns a composition such as described above, comprising an emulsion which contains polyunsaturated fatty acids different from polyunsaturated fatty acids derived from milk and milk proteins in which the majority of fat particles of polyunsaturated fatty acids have a diameter from about 0.21 µm to about 3 µm, particularly from about 0.25 µm to about 3 µm.

The diameter can be expressed by using the Mie-Lorenz theory. The "Mie diameter" or D(4,3) is the volume mean diameter. It is the mean of the diameters of spheres having the same volume as the real particles of the composition of the invention. This mean is analogous to moments of inertia and introduces another linear term in diameter (i.e surface area has a 3D dependence and volume or mass have a D4 dependence as below): D(4,3): (1⁴+2⁴+3⁴)/(1³+2³+3³)=2.72

In the present invention, the composition such as described above, comprises an emulsion which contains polyunsaturated fatty acids different from polyunsaturated fatty acids derived from milk and milk proteins in which the fat particles have a Mie diameter of 0.61 µm.

The composition of the invention can favourably contain vitamin E at a concentration from about 1.5 to about 600mg / 100g.

Vitamin E is the most important lipid-soluble, membrane bound antioxidant in the body and experimental evidence suggests that topical and oral vitamin E have photoprotective and skin-barrier-stabilizing properties (Packer et al., "Molecular aspects of alpha-tocotrienol antioxidant action and cell signalling. J Nutr 2001: 11: 369S-373S).

The composition of the invention is a stable oil-in-water emulsion, comprising polyunsaturated fatty acids and milk proteins in a continuous aqueous phase containing other active substances such as ferments and green tea.

The daily recommendation of this composition intake is 1 or 2 servings depending of the choice of the active ingredients concentrations.

### FIGURES

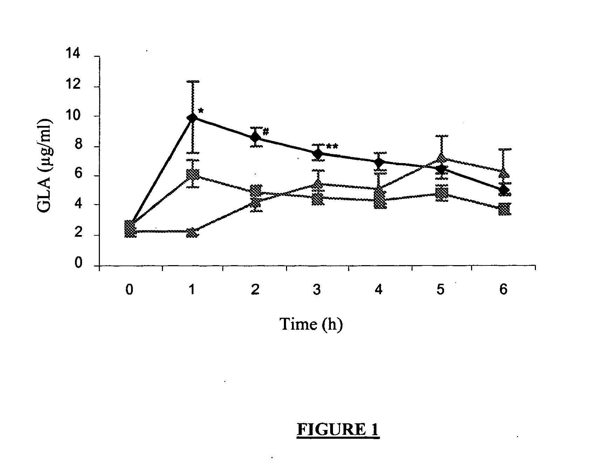

**Figure 1:** Figure 1 represents the average chylomicron concentration of GLA over 6 hours after consumption of dairy products containing borage oil or borage oil alone. The ordinate corresponds to the average chylomicron concentration (in µg/ml) and the abscissa corresponds to the time (in hours). The line with rhombus (◆) corresponds to the dairy product 1 (GLA 300 mg). The line with squares (■) corresponds to the dairy product 2 (GLA 150 mg). The line with triangles (▲) corresponds to the borage oil alone.

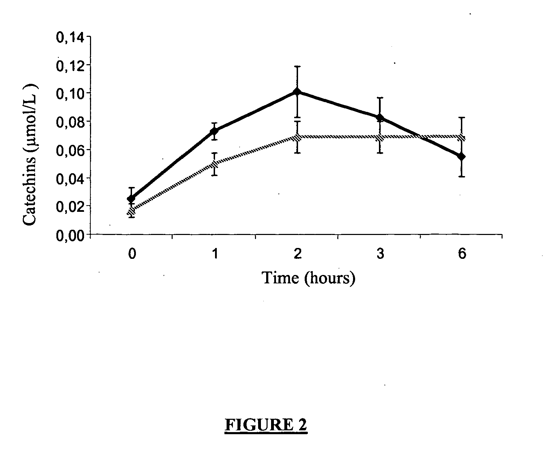

**Figure 2:** Figure 2 represents the plasma concentration of catechins over 6 hours after consumption of dairy products containing green tea extract or green tea extract alone dissolved in 125 ml of water. The ordinate corresponds to plasma concentration of catechins (in µmol/l) and the abscissa corresponds to the time (in hours). The line with rhombus (◆) corresponds to the dairy product 1 (catechins 45 mg + GLA 300 mg in fermented milk matrix). The line with triangles (▲) corresponds to the green tea extract alone, product 3 (see table page 25).

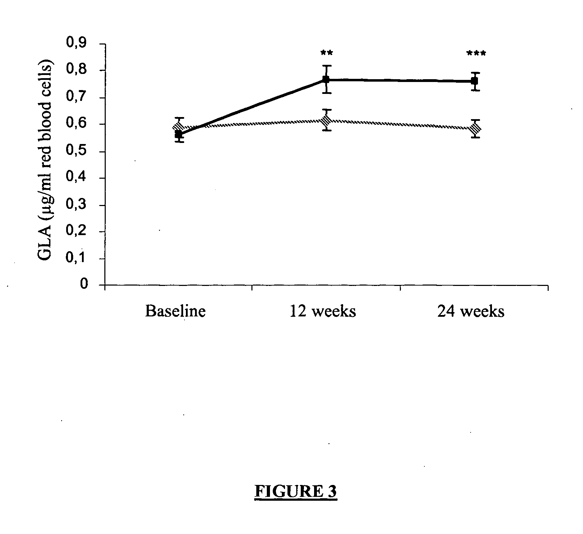

**Figure 3:** Figure 3 represents the GLA concentration in red blood cell lipids (RBCL) over 24 weeks after consumption of a product A corresponding to a fermented dairy product containing probiotics, 30 mg of GLA, 90 mg of catechins and 4 mg of vitamin E or after consumption of a control product (acidified milk). The ordinate corresponds to plasma concentration of GLA in red blood cells (in µg/ml) and the abscissa corresponds to the time (in weeks). The line with squares (■) corresponds to the dairy product A. The line with rhombus (◆) corresponds to the control product.

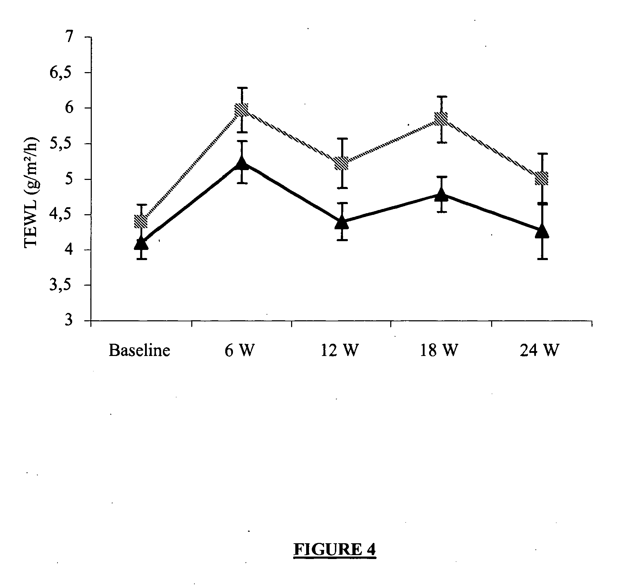

**Figure 4:** Figure 4 represents the transepidermal water loss (TEWL) in Intend To Treat population (ITT population) of two groups at each time points (6, 12, 18, and 24 weeks). The ordinate corresponds to the TEWL values (in g/m²/h) and the abscissa corresponds to the time (in weeks). The line with triangles (▲) corresponds to the test product. The line with squares (■) corresponds to the control product.

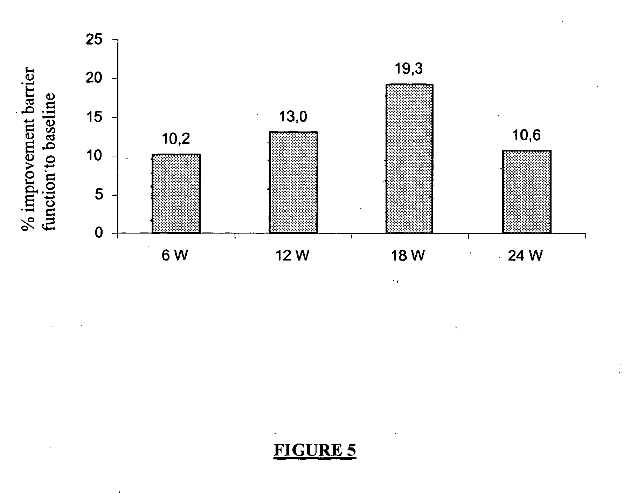

**Figure 5:** Figure 5 represents the percentage of increase of TEWL values in ITT population of two groups at each time points (6, 12, 18, and 24 weeks). The ordinate corresponds to the percentage of increase of TEWL values (% improvement barrier function) compared to base line and the abscissa corresponds to the time of consumption (in weeks).

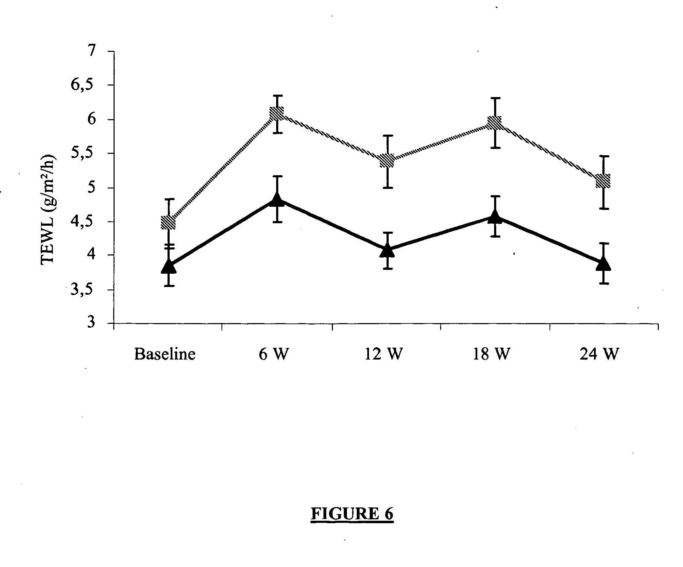

**Figure 6:** Figure 6 represents the transepidermal water loss (TEWL) in the subgroup BMI < 25 (BMI means Body Mass Index expressed in weight (in kg)/height² (in cm)) of two groups at each time points (6, 12, 18, and 24 weeks). The ordinate corresponds to the TEWL values (in g/m²/h) and the abscissa corresponds to the time (in weeks). The line with triangles (▲) corresponds to the test product. The line with squares (■) corresponds to the control product.

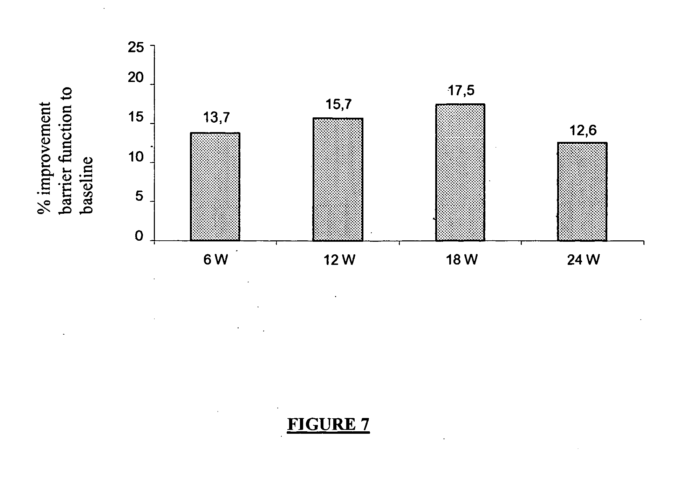

**Figure 7:** Figure 7 represents the percentage of increase of TEWL values in the subgroup BMI < 25 of two groups at each time points (6, 12, 18, and 24 weeks). The ordinate corresponds to the percentage of increase of TEWL values (% improvement barrier function) compared to base line and the abscissa corresponds to the time of consumption (in weeks).

### EXAMPLES

### Example 1: Specification of active ingredients

### Specification of the borage oil

The borage plant is a relatively large plant with star shaped, bright blue flowers and it is found wild in almost all parts of the world (synonym starflower). It grows in South and Central Europe and is cultivated elsewhere. The edible parts of borage plant are leaves, flowers and seeds. It is a well-known herb that has been recognized and used for over 1500 years in food. The borage oil is obtained from the *borago officianalis* seeds. This oil is already used through the Europe as food, food ingredient (for seasonings and salads, as food ingredient in infant formulae), and also as an ingredient for food supplements.

Borage oil is obtained by mechanical pressing in a manner very similar to established processes edible vegetable oil manufacture. It is fully refined (neutralised, bleached, deodorised) using activated carbon. It is a solvent-free process and is not subjected to temperatures above 190°C at any stage of the processing.

Borage oil is a potent Gamma-Linolenic Acid or GLA source, having the highest GLA content of any available oil seed. It is also a significant source of the essential linoleic acid (C18:2 ω-6).

| **Nature of fatty acids** | **Specification (%)** | **Analytical method** |
|---|---|---|
| Palmitic acid (C16:0) | 9-12 | ISO 11885 |
| Palmitoleic acid (C16:1) | < 0.6 | ISO 11885 |
| Stearic acid (C18:0) | 2-6 | ISO 11885 |
| Oleic acid (C18:1) | 12-22 | ISO 11885 |
| Linoleic acid (C18:2 ω-6) | 30-41 | ISO 11885 |
| **Gamma-Linolenic Acid (GLA)**(C18:3 ω-6) | **20-25** | ISO 11885 |
| Alpha-Linolenic Acid (C18:3 ω-3) | ≤0.5 | ISO 11885 |
| Arachidic acid (C20:0) | ≤0.5 | ISO 11885 |
| Eicosenoic acid (C20:1) | 2.8-4.4 | ISO 11885 |
| Erucic acid (C22:1) | ≤3 | ISO 11885 |
| Nervonic acid (C24:1) | ≤4.5 | ISO 11885 |

### Specification of the green tea extract

The green tea extract is made from high quality green tea leaves from the *Camellia sinensis* plant. The manufacturing process corresponds to a regular process used for tea extracts already used as flavouring extracts in Europe (as an example in tea ready to drinks). It is produced by water infusion of the leaves. The extracted solution is filtered, concentrated, purified, decaffeinated using food grade solvent authorised in Europe (e.g. ethyl acetate - Council Directive 88/344/CEE of 13 June 1988) and spray dried.

The green tea extract is mainly composed with polyphenols and specially catechins which belong to the flavan-3-ol class of flavonoids.

The catechin content represents at least 80 % of the extract.

Among catechins, the three following derivatives are main components: (-) epigallocatechin gallate (EGCg), (-)-epigallocatechin (EGC) and (-)-epicatechin (EC) and contains also other catechins as (+)-catechin (C), (+)-gallocatechin (GC), epicatechin gallate (ECg), gallocatechin gallate (GCg).

The green tea extract used in the dairy products corresponds to the following specifications:

| **Test** | **Specification** | **Analytical method** |
|---|---|---|
| EGCg (%) | > 40 | ISO 14502-2 |
| EGC (%) | > 18 | ISO 14502-2 |
| EC (%) | > 8 | ISO 14502-2 |
| Total catechins (%) | > 80 | ISO 14502-2 |
| Total polyphenols (%) | > 80 | ISO 14502-2 |
| Caffein (%) | < 1 | ISO 14502-2 |

### Example 2: Characterization of the emulsion (size and structure)

For the measurement of emulsion, the equipment used is the MASTERSIZER S (MSS) (Malvern). The granulometry has been performed on the emulsions after homogenisation and heat treatment. The granulometry has been performed by dilution with 1% SDS. This technical assay enables to assess the medium diameter of particles by a distribution in volume by diameter of particles.

The transmission electronic microscopy (TEM) allows the structure analysis of emulsion and the observation of fat particles. This methodology is very well known by the skilled person.

The diameter of the majority of fat particles of polyunsaturated fatty acids is from 250 to 3000 nm, with two main populations of particles with a diameter from 500 to 600 nm and a diameter from 1000 to 3000 nm. The lipoproteins interactions are strong; fat particles are related to milk proteins and these proteins surround fat particles.

### Example 3: Bioavailability study of the GLA (gamma-linolenic acid) contained in borage oil and of the green tea catechins.

The study was designed in order to evaluate in a fermented dairy product, the bioavailability of the GLA contained in borage oil and the green tea catechins, by measuring their levels in blood plasma after a single bolus dose. The plasmatic concentrations of GLA and catechins were followed at different times after ingestion.

### Products tested

3 kinds of products were developed and tested:
- Product 1: fermented dairy product (1 serving) containing 3 probiotic strains, GLA (300 mg per serving), catechins (45 mg per serving).
- Product 2: fermented dairy product (1 serving) containing 3 probiotic strains, GLA (150 mg per serving), catechins (45 mg per serving).

Products 1 and 2 were produced following the manufacturing process described in the invention. They were not very acidic (pH 4.52), were creamy and smooth in the mouth. The two products were flavored with a preparation of fruit.
- Product 3 (compounds only): GLA (300 mg), catechins (45 mg).

The borage oil was given directly to the subjects in the form of oil, whereas the green tea extract was dissolved in 125 ml of water.

### General study design

This study was a monocentre, randomised, open, crossover study conducted with 12 healthy female volunteer. The mean age of the population was 30.8 years old and the mean Body Mass Index (BMI) was 21.4. The total duration of the study is around 2 to 3 weeks for each subject.

Each subject has 4 visits of approximately 24 hours each as presented in the planning phase just below:
- an inclusion visit (V1) one week before the first evaluation visit,
- and three evaluation visits : the first visit at J0 (V2), the second at J+4 (V3), the third at J+8 (V4).

Each visit includes a clinical examination. At each of this visit, the subject gave blood to measure GLA and catechins levels and to follow kinetics of the absorption.

After the inclusion visit (V1), the first week was devoted to put into practice the dietary recommendations aimed at reducing the consumption of foods rich in the compounds studied, in order to limit any interference with the compounds tested. For each subject the three evaluation visits were separated by a minimum of 4 days and a maximum of 7 days to have a wash-out period.

### Evaluation visits design

During each visit (V2, V3, V4), the subjects consume one of the products in a randomised fashion, indeed at the end of V4, the subjects consumed the 3 tested products (product 1, 2 and 3 presented above).

Each visit took place as follows and as described in the schema below:
➢ The day before the test visit, after their arrival at the centre at the end of the afternoon, the subjects consumed the dinner offered to them, in a maximum of 30 minutes. The composition of the dinner was identical for all subjects. The dinner was eaten at about 20:00 h.
➢ The day of the test visit, breakfast was served to the volunteers at about 08:00 h, and consumed in a maximum of 20 minutes. Four hours and 30 minutes later, the test lunch was served to the volunteers. It had to be ingested in less than 25 minutes. Twenty five minutes after the start of lunch the volunteers ingested the test product, in less than one minute. The time of start of ingestion of the product was T0 for kinetics. At the end of the kinetics, a dinner was offered to the subjects.

The subjects had 7 blood samples taken in the 6 hours following ingestion of the product according to the above plan. Six sampling times (T0, T1h, T2h, T3h, T4h, T6h) were used to determine both GLA and green tea catechins plasma kinetics. Based on difference of absorption between the ingredients, we have specific time points:
- one timing (T0.5h) was only used to determine the plasma bioavailability of catechins,
- one timing (T5H) was used solely for the determination of the plasma bioavailability of GLA.

### Plasma analysis

The catechins were analysed directly in plasma whereas the GLA measurements were conducted from the chylomicron fraction isolated by ultracentrifugation. The plasma levels of GLA and the plasma levels of the main 3 green tea catechins (epicatechin (EC), epigallocatechin (EGC), and epigallocatechin gallate (EGCg)) were evaluated.

The lipid extraction of the chylomicrons was based on the protocol by Moilanen et al. (Moilanen T., Nikkari T. The effect of storage on the fatty acid composition of human serum. Clin Chim Acta. 1981; 114:111-6). The measurements were done by gas chromatography. The identification of GLA was realized by referring to internal standards. The results are expressed in µg/L.

The plasma concentrations for EC, EGC and EGCg were determined by HPLC as described by Lee et al. (Lee MJ, Prabhu S, Meng X, Li C, Yang CS. An improved method for the determination of green and black tea polyphenols in biomatrices by high-performance liquid chromatography with colorimetric array detection. Anal Biochem. 2000; 279:164-9) and by referring to internal standards. The results are expressed in µmol/L.

### Statistical analysis

The analysed parameters in order to monitor GLA and catechins bioavailability were:
- the "area under the curve" (AUC) that gives information of the kinetics of the bioavailability (µg/mlxh)
- the maximal concentration during the kinetic (C max) (µg/ml)
- the time where the concentration is maximal (T max) (h)

Statistical significance was determined using a Students T-test. The analysis was done on the PP population (Per Protocol, n=11) because one of the 12 included subject did not complete all the visits.

### Results for GLA bioavailability

Two ways of presentation and analysis of the results were done:
a) AUC, Cmax & Tmax: The area under the curve of the absorption kinetics provides information on the overall bioavailability of the ingredients while C max gives the maximal concentration and T max describes the time where the concentration of the ingredient is maximal. The next table summarises the mean between the AUC, Cmax and T max for each product.
b) The kinetics of GLA over 6 hours of consumption for 11 subjects.
a) AUC, Cmax & Tmax analysis are illustrated by the next table.

| | AUC ^{0-6h} (µg/ml/h) | Cmax (µg/ml) | Tmax (h) |
|---|---|---|---|
| **Dairy Product 1** **(GLA 300 mg + Catechins 45 mg in fermented milk matrix)** | 27.9 ± 9.1^{a} | 12.1 ± 7.03^{b} | 2.00 ± 1.00^{c} |
| **Dairy Product 2** **(GLA 150 mg + Catechins 45 mg in fermented milk matrix)** | 12.3 ± 3.1 | 6.5 ± 2.7 | 1.91 ± 1.58^{c} |
| **Dairy Product 3** **(GLA 300 mg alone)** | 15.20 ± 10.50 | 9.4 ± 5.2 | 4.55 ± 1.29 |

| | | | |
|---|---|---|---|
| *Results are mean* ± *SD* *^{a} statistically different from products 2 and 3 (p<0.001); ^{b} statistically different from products 2 (p<0.01);* *^{c} statistically different from product 3 (p<0.01);* | | | |

### > Product 1 vs. 3

The statistical analysis shows important differences between product 1 and 3 (same amount of GLA, either with Fermented Dairy Product (1) or alone as the oil (3)). AUC is significantly higher with product 1 than with product 3, while Cmax is not significantly different. Tmax is significantly delayed with product 3 indicating a slower absorption.

### > Product 1 vs. 2

The results indicate that the amount of GLA absorbed is approximately twice as high with product 1 compared with product 2. This is reflected by both AUC and Cmax, which are significantly lower with product 2.

### > Product 2 vs. 3

The statistical analysis conducted following these observations has shown no significant difference between product 2 and 3, although the ingested amount of GLA is twice lower with product 2 than with product 3. Only Tmax is significantly delayed with product 3 as compared with product 2.

### b) GLA kinetics analysis over 6 hours

A secondary analysis was performed which compared at each time the GLA concentrations in plasma after ingestion of the different products. The Figure 1 summarises the data for the kinetics of each subject.

The statistical analysis shows a significant difference between products 1 and 2, from T 2h to T 3h.

A difference is shown between product 1 and 3 as soon as T 1h. It remains until T 2h. AUC is significantly higher with product 1 than with 3, while Cmax is not significantly different. Tmax is significantly delayed with product 3. The ingested amount of GLA is the same in both products: these results may reflect a better bioavailability when GLA is ingested in fermented dairy product than when it is ingested alone.

There is no difference between product 2 and 3, although the ingested amount of GLA is twice lower with 2 than with 3. This confirms the previous finding.

### c) GLA kinetics analysis in the red blood cell lipids

GLA concentration in the red blood cell lipids (RBCL) was assayed during the study to follow the consumption of the product A corresponding to a fermented dairy product containing probiotics, 30 mg of GLA, 90 mg of catechins and 4 mg of vitamin E. The results demonstrated that GLA accumulated in the RBCL after consumption of the tested product A. An increase of GLA in RBCL was noted in the tested group (product A) *versus* the control group (control product which comprises acidified milk without GLA, catechins, vitamin E and probiotics) as of 12 weeks (p=0.0047) and was prolonged over 24 weeks (p=0.0001). The results are presented in Figure 3. Similar results were obtained for BMI<25 population, with p=0.0003 at 12 weeks and p=0.0005 at 24 weeks after product consumption (not shown).

The accumulation of GLA in RBCL is a good mean of delivering GLA to body tissues, in particular the skin.

### Results for catechin bioavailability

AUC, Cmax & Tmax analysis are illustrated by the next table.

| | AUC ^{0-6h} (µg/ml/h) | Cmax (µg/ml) | Tmax (h) |
|---|---|---|---|
| **Dairy Product 1** **(GLA 300 mg + Catechins 45 mg in fermented milk matrix)** | 0.248 ± 0.047 | 0.098 ± 0.015 | 2.00 ± 1.00 |
| **Dairy Product 3** **(Catechins 45 mg alone)** | 0.310 ± 0.026 | 0.088 ± 0.006 | 4.55 ± 1.29 |

The 3 main catechins were measured in plasma at various time points after ingestion. Concentrations at T0, T2h, T3h and T6h has been analysed in a first time. The Figure 2 summarises the data. The small amount of catechins consumed (45 mg of total catechins) lead to weak plasma concentrations (0.02-0.20 µmol/l) of individual catechins (EC, EGC, EGCG) whatever the consumed product (catechin alone or in the fermented product). These concentrations were at the threshold of quantification by HPLC. Determination of overall catechin concentration did not show any more important changes. The other time points have not been analysed.

### Conclusion:

Pharmacokinetic parameters in chylomicrons show that GLA from borage oil, at 150 and 300 mg/pot, in fermented dairy products tested is found in plasma.

Significantly different responses on AUC and Cmax for GLA in chylomicrons were observed after consumption of the two dairy products. The absorbed quantity (e.g. AUC and Cmax) is 2-fold higher for dairy product with 300 mg of GLA than for dairy product with 150 mg of GLA. The dose response effect is found for these GLA concentrations.

The GLA absorption kinetic is different in dairy products from borage oil alone (Tmax shorter in dairy products). AUC after consumption of dairy product with 300 mg of GLA is significantly higher than borage oil alone containing also 300 mg of GLA. The GLA quantities found in the plasma (e.g. AUC and Cmax) after a dairy product intake (with 150 mg of GLA) or alone borage oil intake (300 mg of GLA) are similar. The incorporation of borage oil into a dairy substance leads to a positive effect on the absorption of GLA by increasing the levels of the GLA in the chylomicrons lipoprotein fraction in the plasma. This can explain that higher concentrations of GLA can reach the skin to exert their beneficial effect on skin.

The catechin results show that consumption of 45 mg of catechins from green tea extract either in fermented dairy product or alone lead to small increases of plasma concentrations of some individual catechins. No difference in the catechins absorption kinetic was detected after consumption of the two dairy products or green tea extract alone. The weak plasma concentrations seem to be in agreement with the literature data. Moreover, it is known that some catechins metabolites could act on the skin but their precise nature/function are not documented in the literature. Based in this statement, the catechins are maintained in the same level in our product by postulating an additional effect with other ingredients.

### Example 4: Clinical study

This study was a single-centre, randomised, double-blinded, parallel study in healthy female volunteers with dry and sensitive skin. Women were chosen as these are the target population and virtually all of cosmetic products are tested on women. 72 subjects were divided into 2 balanced groups of 36 subjects with a mean age 29.4 ± 7.9 and BMI mean 22.43 ± 2.8. The subjects have received diet recommendation and no cosmetics use before the beginning of the study and during all the study.

One group received the test product which comprises 150 mg GLA, 47 mg catechins and 2 mg vitamin E, mixed in a dairy matrix containing probiotic (*Lactobacillus casei, bulgaricus and Streptococcus thermophilus*) (test group) and the other received the control product which comprises acidified milk without GLA, catechins, vitamin E and probiotics (control group). The study was divided in two parts, the selection phase (4 weeks) and the consumption phase (24 weeks). The subjects attended 4 evaluation visits 6 weeks apart during the period of the consumption of the product (consumption of the product (test or control) at two pots/day). A six month period of testing allowed the comparison of the effect of the product over an extended period of time and allowed an evaluation of its effects in different seasons of the year.

The tested product was compared to a control product without probiotic, borage oil and catechins to allow comparison of the effects of these ingredients on skin functionality. The barrier function of the skin was assessed by transepidermal water loss (TEWL) which is an accepted method of measuring the barrier function of the stratum corneum (SC). The TEWL was measured on the inner forearm with a Servomed EP2^{®} "EVAPORIMETER". The study was initiated in autumn through to spring to compare the effects of the product on skin functioning at different times of the year; the skin's barrier function is known to deteriorate in the winter months of the year. Analysis of the data was done on the total subjects and also on different sub groups defined at the beginning of the study and based on the inclusion criteria (BMI and hydration level).

The inventors analysed the data in 2 ways:
- comparison between the 2 groups at each time point (6, 12, 18 and 24 weeks). The inventors analysed with a covariance analysis of the raw changes;
- comparison of the "evolution" of the effect during the time; this more global analysis on the kinetics of the response allows to take into account the difference between the 2 products and the variation due to the environment, they used a mixed model on repeated measurements on 2 periods on time: the total study (24 weeks) and until 12 weeks (primary criteria of the study).

These analyses take into account the baseline levels in each case.

### Results on ITT population

The inventors analysed the ITT population (Intend To Treat population, n=67). As can be observed in the Figure 4, there is an improvement in barrier function relative to the control (lower TEWL in product group) at all time points following consumption of the product. The analysis of evolution during the time reveals that the tested product reduces significatively the TEWL during all the study (24 weeks consumption (p=0.026)) but also on the phase until 12 weeks consumption (p=0.036). Moreover, on the total period of the study (24 weeks), the adjusted mean difference on the TEWL values between the groups is 0.68 g/m²/h.

There is a natural variation in barrier function (i.e. a slight deterioration at winter reflected by a TEWL increased value) but the active product shows a trend of superiority to control at 6 weeks and at 18 weeks the differences are statistically significant.

If we compared the relative percentage of increase of TEWL values in the 2 groups (see Figure 5), we can notice that the highest difference is observed at 18 weeks with 19.3% between the 2 groups from baseline.

On average, on all the period of the study, the difference on the TEWL between the 2 groups is 13.25%. That's mean that the product retains about 14% more water than the control.

### Results on the sub group BMI < 25

The classification on BMI gave interesting results. The analysis in the sub group "BMI<25" (n=53) reveals a higher effect of the product.

When the inventors compared the evolution of the 2 groups during the study, they saw that there is a significant difference in the product during the period until 24 weeks (p=0.005) (see Figure 6).

In this population also, there is a defective epidermal skin barrier function due to winter (reflected by a TEWL increased values) but the product shows a significant superiority to control on all the duration of the study (p= 0.005) to avoid excessive water loss.

Moreover, on the total period of the study (24 weeks), the mean difference on the TEWL values between the groups is 0.95 g/m²/h.

When the inventors compare the percentage of TEWL increase between the 2 groups (see Figure 7), they showed 15.7% and 17.5% improvements in barrier function relative to control respectively as soon as after 6 weeks of consumption and 18 weeks.

On average, on all the period of the study, the difference on the TEWL between the 2 groups is 14.9%. That means that the product retains about 15% more water than the control.

### Conclusion:

Relative to control, skin barrier function was significantly improved after consumption of a composition comprising, as active substance, a mixture of at least one polyunsaturated fatty acid, different from polyunsaturated fatty acid derived from milk, polyphenols, milk proteins and lactic bacteria, and said composition having a water content of at least 50% by weight. The skin was better able to resist the environment impact on barrier function presumably due to changes in *stratum corneum* composition. The reinforcement of barrier function was probably due to combined effects of GLA, catechins, probiotics and optionally vitamin E on epidermal differentiation.

## Claims

1. A composition comprising, as active substance, a mixture of at least gamma-linolenic acid, polyphenols, milk proteins and lactic bacteria, and said composition having a water content of at least 50% by weight, and wherein the said gamma-linolenic acid presents an increased bioavailability once ingested compared with the one of the gamma-linolenic acid alone.

2. A composition comprising, as active substance, a mixture of at least one polyunsaturated fatty acid, different from polyunsaturated fatty acid derived from milk, polyphenols, milk proteins and lactic bacteria, and said composition having a water content of at least 50% by weight, and wherein the concentration of polyphenols represents 0.13% to 18.7%, particularly 0.15% to 15%, and more particularly 0.16% to 13% of the total weight of the active substance, and wherein the said polyunsaturated fatty acid presents an increased bioavailability once ingested compared with the one of the polyunsaturated fatty acid alone.

3. The composition according to claim 1 or 2, wherein the polyunsaturated fatty acid is contained in one at least of the following vegetable oils: borage oil, primrose oil, black currant oil, camelina oil, hemp seed oil, kiwi seed oil, flax oil, rapeseed oil and micro algae oil, or is produced by a enzymatic process.

4. The composition according to claim 2 or 3, wherein the polyunsaturated fatty acid is selected among linolenic acids, their derivatives and their precursors, and is in particular gamma-linolenic acid.

5. The composition according to any of claims 1 to 4, wherein polyphenols, are contained in a vegetal extract such as green tea extract, grape seed extract, grape skin extract, grapefruit, (red) orange extract, pine bark extract (pycnogenol), red wine extract, tomato extract, gingko biloba extract, cranberry extract, soy extract, soy isoflavons extract, blueberry, raspberry, elderberry, pomegranate, apple, cider, cocoa, green coffee, mango, cactus pear, black currant, seaweeds, olive, cinnamon, ginger, ginseng, aloe vera, brocoli, hibiscus.

6. The composition according to any of claims 1 to 5, wherein polyphenols are selected among: phenolic acids, such as hydroxycinnamic acids, hydroxybenzoic acids; flavonoids such as flavonols, flavones, isoflavones, flavanones, anthocyanidins, proanthocyanidins; hydrolyzable tannins such as gallotannins, ellagitannins and in particular tea catechins, resveratrol, viniferins, caffeic acid, gallic acid, ellagic acid, ferulic acid, chlorogenic acid, quercetin, kaempferol, apigenin, daidzein, genistein, naringenin, hesperidin, anthocyanin, catechin, epicatechin, gallocatechin.

7. The composition according to any of claims 1 to 6 , wherein polyphenols are selected among tea catechins.

8. The composition according to any of claims 1 to 7, wherein at least 50% (w/w), more particularly at least 60% (w/w) of tea catechins are selected among epicatechin, epigallocatechin, epigallocatechin gallate.

9. The composition according to any of claims 1 to 8, wherein milk proteins are contained in one at least of the following dairy substance: milk, skimmed milk, milk powder, skimmed milk powder, cream, milk protein concentrate, whey, whey protein concentrate, and caseinate.

10. The composition according to any of claims 1 to 9, wherein lactic bacteria are chosen among one at least of the following bacteria: *Streptococcus thermophilus, Lactobacillus delbrueckii ssp. bulgaricus, Lactobacillus casei, Lactobacillus paracasei, Lactobacillus acidophilus, Lactobacillus helveticus, Lactobacillus plantarum, Lactobacillus reuteri, Lactobacillus kefiranofaciens, Bifidobacterium lactis ssp animalis, Bifidobacterium longum, Bifidobacterium breve, Lactococcus lactis ssp cremoris, Lactococcus lactis ssp lactis* and *Leuconostoc.*

11. The composition according to any of claims 1 to 10, wherein lactic bacteria are particularly *Streptococcus thermophilus, Lactobacillus bulgaricus* and *Lactobacillus casei.*

12. The composition according to any of claims 1 to 11, wherein the water content is from 70% to 90 %, particularly 75% to 85 %, more particularly 80 % by weight of the composition.

13. The composition according to any of claims 2 to 12, wherein,
- polyunsaturated fatty acids represent 2.3% to 91.5%, particularly 2.3% to 70% and more particularly 2.3% to 65% of the total weight of the active substance;
- polyphenols represent 0.13% to 18.7%, particularly 0.15% to 15%, and more particularly 0.16% to 13% of the total weight of the active substance;
- milk proteins represent 8% to 97.6%, particularly 15% to 97.6% and more particularly 30% to 97.4% of the total weight of the active substance;
- lactic bacteria are present in the active substance in an amount of 10⁶ to 10¹⁰ cfu/mg, particularly 10⁷ to 10⁹ cfu/mg.

14. The composition according to any of claims 2 to 13, wherein the concentration of polyunsaturated fatty acids is from 0.15% to 16.6 %, particularly from 0.2% to 10% and more particularly from 0.4% to 5% by weight of the composition.

15. The composition according to any of claims 1 to 14, wherein the concentration of gamma-linolenic acid is from 0.02% to 1%, particularly from 0.04% to 0.6% and more particularly from 0.1% to 0.2% by weight of the composition.

16. The composition according to any of claims 1 to 15, wherein the concentration of milk proteins is from 1.5% to 6%, particularly from 2.5% to 5% by weight of the composition.

17. The composition according to any of claims 3 to 16, wherein the concentration of the vegetable oil, in particular borage oil, is from 0.2% to 3% by weight of the composition.

18. The composition according to any of claims 5 to 17, wherein the concentration of the vegetal extract, in particular green tea extract, is from 0.01% to 1% by weight of the composition.

19. The composition according to any of claims 3 to 18 comprising borage oil, green tea extract, milk and lactic bacteria, and said composition having a water content of at least 30% by weight, and wherein the polyunsaturated fatty acid contained in borage oil, presents an increased bioavailability compared with the one of the polyunsaturated fatty acid alone.

20. The composition according to any of claims 1 to 19, which is a food or a beverage or a supplement composition for a food or a beverage.

21. The composition according to any of claims 1 to 20, **characterised in that** the food is selected among a fermented dairy product containing at least 50% of the fermented product derived from the dairy substance, a yoghurt and a fruit yoghurt.

22. The non-therapeutic use of a composition according to any of claims 1 to 21, to improve skin quality by acting on skin barrier function or skin moisturizing barrier.

23. A process for preparing a composition comprising, as active substance, a mixture of at least one polyunsaturated fatty acid different from polyunsaturated fatty acid derived from milk, polyphenols, milk proteins and lactic bacteria and having a water content of at least 50% by weight; and wherein the concentration of polyphenols represents 0.13% to 18.7%, particularly 0.15% to 15%, and more particularly 0.16% to 13% of the total weight of the active substance, and wherein the said polyunsaturated fatty acid presents an increased bioavailability compared with the one of the polyunsaturated fatty acid alone,
said process comprising the addition of a vegetal extract containing polyphenols to a fermented homogenized mixture of a vegetable oil containing polyunsaturated fatty acids or enzymatically derived polyunsaturated fatty acids and of a dairy substance containing milk proteins.

24. The process according to claim 23, comprising the preparation of a fermented homogenized mixture of a vegetable oil containing polyunsaturated fatty acids or enzymatically derived polyunsaturated fatty acids and of a dairy substance containing milk proteins by:
- a step of incorporation of a vegetable oil or enzymatically derived polyunsaturated fatty acids within a dairy substance to obtain a mixture of a vegetable oil or enzymatically derived polyunsaturated fatty acids and of a dairy substance;
- a step of homogenisation of the above-mentioned mixture to obtain a homogenized mixture of a vegetable oil or enzymatically derived polyunsaturated fatty acids and of a dairy substance;
- a step of fermentation of the above-mentioned homogenized mixture to obtain a fermented homogenized mixture of a vegetable oil or enzymatically derived polyunsaturated fatty acids and of a dairy substance.

25. A composition obtainable by the process according to claim 23 or 24.

26. The composition according to any of claims 1 to 21 or 25, comprising vitamin E at a concentration from 1,5 to 600mg / 100g.

## Patentansprüche

1. Eine Zusammensetzung, die als Aktivsubstanz eine Mischung von zumindest Gamma-linolensäure, Polyphenolen, Milchproteinen und Milchsäurebakterien enthält, worin diese Zusammensetzung einen Wasserinhalt von zumindest 50 Gew. % hat, und worin diese Gamma-linolensäure eine erhöhte Bioverfügbarkeit nach Nahrungsaufnahme im Vergleich zu der der Gamma-linolensäure allein zeigt.

2. Eine Zusammensetzung, die als Aktivsubstanz eine Mischung von zumindest einer ungesättigten Fettsäure enthält, die anders als polyungesättigte, von Milch, Polyphenolen, Milchproteinen und Milchsäurebakterien abgeleiteten Fettsäure ist, und worin diese Zusammensetzung einen Wasserinhalt von zumindest 50 Gew. % besitzt, und worin die Polyphenol-Konzentration 0,13 bis 18,7 %, insbesondere 0,15 bis 15 %, und ganz besonders 0,16 bis 13 % des ganzen Gewichts der Aktivsubstanz ist, und worin die obengenannte polyungesättigte Fettsäure eine erhöhte Bioverfügbarkeit nach Nahrungsaufnahme im Vergleich zu der der polyungesättigten Säure aufweist.

3. Eine Zusammensetzung nach Anspruch 1 oder 2, worin die polyungesättigte Fettsäure in zumindest einem der folgenden pflanzlichen Ölen enthaltet ist : Borretschöl, Erd-Schlüsselblumenöl, Johannisbeerenöl, Camelinaöl, Hanföl, Kiwisamenöl, Leinöl, Rapsöl und Mikroalgenöl, oder mit einem enzymatischen Verfahren produziert wird.

4. Eine Zusammensetzung nach Anspruch 2 oder 3, worin die polyungesättigte Fettsäure unter Linolensäuren, ihre Derivaten und Vorläufer gewählt wird, und ist insbesondere die Gamma-linolensäure.

5. Die Zusammensetzung nach irgendwelchem Anspruch 1 - 4, worin Polyphenole in einem pflanzlichen Extrakt, wie grüner Teeextrakt, Traubenkernextrakt, Traubenhautextrakt, Pampelmuse, (roter) Orangenextrakt, Kieferrindenextrakt (Pycnogenol), Rotweinextrakt, Tomatenextrakt, Gingko biloba Extrakt, Preiselbeerenextrakt, Sojabohnenextrakt, Soja-Isoflavonenextrakt, Heidelbeerenextrakt, Himbeerenextrakt, Holunderbeerenextrakt, Granatapfel, Apfel, Apfelwein, Kakao, grüner Kaffee, Mangofrucht, Kaktusbirne, schwarze Johannisbeere, Meeresalgen, Oliven, Zimt, Ingwer, Ginseng, Aloe vera, Brokkoli, Hibiscus beinhaltet sind.

6. Die Zusammensetzung nach irgendwelchem Anspruch 1 - 5, worin die Polyphenole unter Phenolsäuren, wie Hydroxyzimtsäuren, Hydroxybenzoesäuren, Flavonoide wie Flavonole, Flavone, Isoflavone, Flavanone, Anthocyanidine, Proanthocyanidine ; hydrolysierbare Tannine wie Gallotannine, Ellagitannine und insbesondere Teekatechine, Resveratrol, Viniferine, Kaffeesäure, Gallussäure, ellagische Säure, ferulische Säure, chlorogenische Säure, Querzetin, Kämpferol, Apigenin, Daidzein, Genistein, Naringenin, Hesperidin, Anthocyanin, Catechin, Epicathechin, Gallocatechin, gewählt werden.

7. Die Zusammensetzung nach irgendwelchem Anspruch 1 - 6, worin die Polyphenole unter Teecatechine gewählt werden.

8. Die Zusammensetzung nach irgendwelchem Anspruch 1 - 7, worin zumindest 50 % (Gew/Gew), und insbesondere zumindest 60 % (Gew/Gew) der Teecatechinen unter Epicatechin, Epigallocatechin, Epigallocatechin-Gallat gewählt werden.

9. Die Zusammensetzung nach irgendwelchem Anspruch 1 - 8, worin die Milchproteine zumindest in einer der folgenden Molkereisubstanzen beinhaltet sind : Milch, Magermilch, Milchpulver, Magermilchpuder, Rahm, Milchproteinkonzentrat, Molke, Molkeproteinkonzentrat und Caseinat.

10. Die Zusammensetzung nach irgendwelchem Anspruch 1 - 9, worin Lactobakterien unter zumindest einem der folgenden Bakterien gewählt werden : *Streptococcus thermophilus, Lactobacillus delbrueckii ssp. bulgaricus, Lactobacillus casei, Lactobacillus paracasei, Lactobacillus acidophilus, Lactobacillus helveticus, Lactobacillus plantarum, Lactobacillus reuteri, Lactobacillus kefiranofaciens, Bifidobacterium lactis ssp. animalis, Bifidobacterium longum, Bifidobacterium breve, Lactococcus lactis ssp. cremoris, Lactococcus lactis ssp. lactis* und *Leuconostoc.*

11. Die Zusammensetzung nach irgendwelchem Anspruch 1 - 10, worin die Lactobacterien insbesondere *Streptococcus thermophilus, Lactobacillus bulgaricus* und *Lactobacillus casei* sind.

12. Die Zusammensetzung nach irgendwelchem Anspruch 1 - 11, worin der Wassergehalt zwischen 70 % und 90 %, insbesondere zwischen 75 und 85 % und ganz insbesondere bei 80 % der Zusammensetzung liegt.

13. Die Zusammensetzung nach irgendwelchem Anspruch 2 -12, worin die polyungesättigte Säuren 2,3 % bis 91,5 %, insbesondere 2,3 % bis 70 %, und ganz insbesondere 2,3 bis 65 % des gesamten Aktivsubstanzgewichts ; die Polyphenole 0,13 % bis 18,7 %, insbesondere 0,15 % bis 15 % und ganz insbesondere 0,16 % bis 13 % des gesamten Aktivsubstanzgewichts ; die Milchproteine 8 % bis 97,6 %, insbesondere 15 % bis 97,6 % und ganz insbesondere 30 % bis 97,4 % des gesamten Aktivsubstanzgewichts bilden ; die Laktobakterien im Betrag von 10⁶ bis 10¹⁰ cfu/mg, insbesondere 10⁷ bis 10⁹ cfu/mg enthalten sind.

14. Die Zusammensetzung nach irgendwelchem Anspruch 2 -13, worin die Konzentration der polyungesättigten Säuren zwischen 0,15 Gew. % und 16,6 Gew. %, insbesondere zwischen 0,2 Gew. % und 10 Gew. %, und ganz insbesondere zwischen 0,4 Gew. % und 5 Gew. % der Zusammensetzung liegt.

15. Die Zusammensetzung nach irgendwelchem Anspruch 1 - 14, worin die Konzentration der Gamma-linolensäure zwischen 0,02 Gew. % und 1 Gew. %, insbesondere zwischen 0,04 Gew. % und 0,6 Gew. %, und ganz insbesondere zwischen 0,1 Gew. % und 0,2 Gew. % der Zusammensetzung liegt.

16. Die Zusammensetzung nach irgendwelchem Anspruch 1 - 15, worin die Konzentration der Milchproteinen zwischen 1,5 Gew. % und 6 Gew. %, insbesondere zwischen 2,5 Gew. % bis 5 Gew. % der Zusammensetzung liegt.

17. Die Zusammensetzung nach irgendwelchem Anspruch 3 - 16, worin die Konzentration des Pflanzenöls, insbesondere des Borretschöls, zwischen 0,2 Gew. % und 3 Gew. % der Zusammensetzung liegt.

18. Die Zusammensetzung nach irgendwelchem Anspruch 5 -17, worin die Konzentration des Pflanzenextrakts, insbesondere des grünen Teeextrakts, zwischen 0,01 Gew. % und 1 Gew. % der Zusammensetzung liegt.

19. Die Zusammensetzung nach irgendwelchem Anspruch 3 - 18, die Borretschöl, grüner Teeextrakt, Milch und Laktobakterien beinhaltet, und worin besagte Zusammensetzung einen Wassergehalt von zumindest 50 Gew. % besitzt, und worin die polyungesättigte Säure, die im Borretschöl beinhaltet ist, eine erhöhte Bioverfügbarkeit im Vergleich mit der der polyungesättigten Säure allein hat.

20. Die Zusammensetzung nach irgendwelchem Anspruch 1 - 19, die eine Nahrung oder ein Getränk, oder eine Ergänzungszusammensetzung für Nahrung oder Getränk ist.

21. Die Zusammensetzung nach irgendwelchem Anspruch 1 - 20, **dadurch gekennzeichnet, daß** die Nahrung unter gegärten Molkereiprodukten gewählt wird, worin zumindest 50 % des gegärten Produkt aus der Molkereisubstanz, einem Joghurt oder einem Fruchtjoghurt abgeleitet ist.

22. Nicht-therapeutische Verwendung einer Zusammensetzung nach irgendwelchem Anspruch 1 - 21, zu Erhöhung der Hautqualität durch Handlung der Sperrfunktion der Haut oder der feuchtigmachenden Sperre der Haut.

23. Ein Verfahren für die Herstellung einer Zusammensetzung, die als Aktivsubstanz eine Mischung von zumindest einer polyungesättigten Fettsäure enthält, die anders als eine aus Milch, Polyphenolen, Milchproteinen und Laktobakterien abgeleitete polyungesättigte Fettsäure ist, und die einen Wassergehalt von zumindest 50 Gew. % hat; und worin die Polyphenolkonzentration zwischen 0,13 Gew. % und 18,7 Gew. %, insbesondere zwischen 0,15 Gew. % und 15 Gew. % und ganz insbesondere zwischen 0,16 Gew. % und 13 Gew. % der Aktivsubstanz liegt, und worin die besagte polyungesättigte Fettsäure eine erhöhte Bioverfügbarkeit im Vergleich mit der der polyungesättigten Fettsäure allein zeigt, worin das besagte Verfahren die Zugabe eines Pflanzenextrakt, das Polyphenolen enthält, zu einer gegärten homogenen Mischung eines Pflanzenöl, das polyungesättigte Fettsäuren oder mit Enzymen abgeleitete polyungesättigte Fettsäuren, und einer Milchproteine enthaltende Molkereisubstanz beinthaltet.

24. Ein Verfahren nach Anspruch 23 die die Bereitung einer gegärten homogenen Mischung eines Pflanzenöl, das polyungesättigte Fettsäuren oder mit Enzymen abgeleitete polyungesättigte Fettsäuren, und einer Milchproteine enthaltende Molkereisubstanz beinthaltet, und zwar mit:
- einem Einfügungsschritt für ein pflanzliches Öl oder mit Enzymen abgeleitete polyungesättigte Fettsäuren innerhalb einer Molkereisubstanz ;
- einem Homogenisierungsschritt für die obengenannte Mischung, und zwar um eine homogenisierte Mischung von einem Pflanzenöl oder von mit Enzymen abgeleiteten polyungesättigten Fettsäuren mit einer Molkereisubstanz zu erhalten ;
- einem Gärungsschritt der obengenannten homogenisierten Mischung, und zwar um eine homogenisierte gegärte Mischung von einem Pflanzenöl oder von mit Enzymen abgeleiteten polyungesättigten Fettsäuren mit einer Molkereisubstanz zu erhalten.

25. Eine Zusammensetzung, die mit dem Verfahren nach Anspruch 23 oder Anspruch 24 erhältlich ist.

26. Die Zusammensetzung nach irgendwelcher Anspruch 1 bis 21 oder 25, die Vitamin E bei einer Konzentration zwischen 1,5 und 600 mg / 100 g beinhaltet.

## Revendications

1. Composition comprenant, comme substance active, un mélange d'au moins de l'acide gamma-linolénique, des polyphénols, des protéines de lait et des bactéries lactiques, et ladite composition ayant une quantité d'eau d'au moins 50% en poids, et dans laquelle ledit acide gamma-linolénique présente une biodisponibilité augmentée, une fois ingéré, comparée à celle de l'acide gamma-linolénique seul.

2. Composition comprenant comme substance active un mélange d'au moins un acide gras poly-insaturé, différent d'un acide gras poly-insaturé dérivé du lait, des polyphénols, des protéines de lait et des bactéries lactiques, et ladite composition ayant une quantité d'eau d'au moins 50% en poids, et dans laquelle la concentration en polyphénols représente de 0,13% à 18,7%, particulièrement de 0,15% à 15%, et plus particulièrement de 0,16% à 13% du poids total de la substance active, et dans laquelle ledit acide gras poly-insaturé présente une biodisponibilité augmentée, une fois ingéré, comparée à celle de l'acide gras poly-insaturé seul.

3. Composition selon la revendication 1 ou 2, dans laquelle l'acide gras poly-insaturé est contenu dans au moins une des huiles suivantes : huile de bourrache, huile d'onagre, huile de cassis, huile de caméline, huile de graine de chanvre, huile de graine de kiwi, huile de lin, huile de graine de colza et huile de micro algues, ou est produit par un procédé enzymatique.

4. Composition selon la revendication 2 ou 3, dans laquelle l'acide gras poly-insaturé est choisi parmi les acides linoléniques, leurs dérivés et leurs précurseurs et est en particulier de l'acide gamma-linolénique.

5. Composition selon l'une des revendications 1 à 4, dans laquelle les polyphénols sont contenus dans un extrait végétal tel que de l'extrait de thé vert, de l'extrait de pépin de raisin, de l'extrait de peau de raisin, du raisin, de l'extrait d'orange (sanguine), de l'extrait d'écorce de pin (pycnogenol), de l'extrait de vin rouge, de l'extrait de tomate, de l'extrait de ginkgo biloba, de l'extrait de canneberge, de l'extrait de soja, de l'extrait d'isoflavones de soja, de la myrtille, de la framboise, du sureau, de la grenade, de la pomme, du cidre, du cacao, du café vert, de la mangue, figue de barbarie, du cassis, de l'algue marine, de l'olive, de la cannelle, du gingembre, du ginseng, de l'aloe vera, du brocoli, de l'hibiscus.

6. Composition selon l'une des revendications 1 à 5, dans laquelle les polyphénols sont choisis parmi : les acides phénoliques tels que les acides hydroxycinnamiques, les acides hydroxybenzoïques, les flavonoïdes tels que les flavonols, les flavones, les isoflavones, les flavonones, les anthocyanidines, les proanthocyanidines, les tannins hydrolysables tels que les gallotannins, les ellagitannins et en particulier, les catéchines de thé, le resveratrol, les viniférines, l'acide caféique, l'acide gallique, l'acide ellagique, l'acide férulique, l'acide chlorogénique, la quercétine, le kaempférol, l'apigénine, la daidzéine, la génistéine, la naringinine, l'hespéridine, l'anthocyanine, la catéchine, l'épicatéchine, la gallocatéchine.

7. Composition selon l'une des revendications 1 à 6, dans laquelle les polyphénols sont choisis parmi les catéchines du thé.

8. Composition selon l'une des revendications 1 à 7, dans laquelle au moins 50% (m/m), plus particulièrement au moins 60% (m/m) des catéchines de thé sont choisies parmi l'épicatéchine, l'épigallocatéchine, le gallate d'épigallocatéchine.

9. Composition selon l'une des revendications 1 à 8, dans laquelle des protéines de lait sont contenues dans au moins une des substances laitière suivantes : le lait, le lait écrémé, le lait en poudre, le lait écrémé en poudre, la crème, le concentré de protéines de lait, le lactosérum, le concentré de protéine de lactosérum et la caséine.

10. Composition selon l'une des revendications 1 à 9, dans laquelle les bactéries lactiques sont choisies parmi une au moins des bactéries suivantes *Streptococcus thermophilus, Lactobacillus delbrueckii ssp. bulgaricus, Lactobacillus casei, Lactobacillus paracasei, Lactobacillus acidophilus, Lactobacillus helveticus, Lactobacillus plantarum, Lactobacillus reuteri, Lactobacillus kefiranofaciens, Bifidobacterium lactis ssp. animalis, Bifidobacterium longum, Bifidobacterium breve, Lactococcus lactis ssp. cremoris, Lactococcus lactis ssp. lactis* et *Leuconostoc.*

11. Composition selon l'une des revendications 1 à 10, dans laquelle les bactéries lactiques sont particulièrement : *Streptococcus thermophilus, Lactobacillus bulgaricus* et *Lactobacillus casei.*

12. Composition selon l'une des revendications 1 à 11, dans laquelle la quantité d'eau est de 70% à 90%, particulièrement 75% à 85%, plus particulièrement 80% en poids de la composition.

13. Composition selon l'une des revendications 2 à 12, dans laquelle :
- les acides gras poly-insaturés représentent de 2,3% à 91,5%, particulièrement de 2,3% à 70% et plus particulièrement de 2,3% à 65% du poids total de la substance active;
- les polyphénols représentent de 0,13% à 18,7%, particulièrement de 0,15% à 15%, et plus particulièrement de 0,16% à 13% du poids total de la substance active;
- les protéines de lait représentent de 8% à 97,6%, particulièrement de 15% à 97,6% et plus particulièrement de 30% à 97,4% du poids total de la substance active;
- les bactéries lactiques sont présentes dans la substance active en quantité de 10⁶ à 10¹⁰ cfu/mg, particulièrement de 10⁷ à 10⁹ cfu/mg.

14. Composition selon l'une des revendications 2 à 13, dans laquelle la concentration en acides gras poly-insaturés est de 0,15% à 16,6 %, particulièrement de 0,2% à 10% et plus particulièrement de 0,4% à 5% en poids de la composition.

15. Composition selon l'une des revendications 1 à 14, dans laquelle la concentration en acide gamma-linolénique est de 0,02% à 1 %, particulièrement de 0,04% à 0,6% et plus particulièrement de 0,1% à 0,2% en poids de la composition.

16. Composition selon l'une des revendications 1 à 15, dans laquelle la concentration en protéines de lait est de 1,5% à 6%, particulièrement de 2,5% à 5% en poids de la composition.

17. Composition selon l'une des revendications 3 à 16, dans laquelle la concentration en huile végétale, en particulier en huile de bourrache, est de 0,2% à 3% en poids de la composition.

18. Composition selon l'une des revendications 5 à 17, dans laquelle la concentration de l'extrait végétal, en particulier d'extrait de thé vert, est de 0,01% à 1% en poids de la composition.

19. Composition selon l'une des revendications 3 à 18, comprenant de l'huile de bourrache, de l'extrait de thé vert, du lait et des bactéries lactiques, et ladite composition ayant une quantité d'eau d'au moins 50% en poids, et dans laquelle l'acide gras poly-insaturé contenu dans l'huile de bourrache, présente une biodisponibilité augmentée comparée à celle de l'acide gras poly-insaturé seul.

20. Composition selon l'une des revendications 1 à 19, qui est une composition alimentaire ou à boire ou de complément, pour un aliment ou une boisson.

21. Composition selon l'une des revendications 1 à 20, **caractérisée en ce que** l'aliment est choisi parmi un produit laitier fermenté contenant au moins 50% de produit fermenté dérivé d'une substance laitière, un yoghourt et un yoghourt aux fruits.

22. Utilisation non thérapeutique d'une composition selon l'une des revendications 1 à 21, pour améliorer la qualité de la peau en agissant sur la fonction de barrière de la peau ou de barrière d'humidité de la peau.

23. Procédé pour la préparation d'une composition comprenant, comme substance active, un mélange d'au moins un acide gras poly-insaturé différent d'un acide gras poly-insaturé dérivé du lait, des polyphénols, des protéines de lait et des bactéries lactiques et ayant une quantité d'eau d'au moins 50% en poids; et dans lequel la concentration en polyphénols représente de 0,13% à 18,7%, particulièrement de 0,15% à 15%, et plus particulièrement de 0,16% à 13% du poids total de la substance active, et dans lequel ledit acide gras poly-insaturé présente une biodisponibilité augmentée comparée à celle de l'acide gras poly-insaturé seul,
ledit procédé comprenant l'addition d'un extrait végétal contenant des polyphénols à un mélange homogénéisé fermenté d'une huile végétale contenant des acides gras poly-insaturés ou des acides gras poly-insaturés dérivés de manière enzymatique et d'une substance laitière contenant des protéines de lait.

24. Procédé selon la revendication 23, comprenant la préparation d'un mélange homogénéisé fermenté d'une huile végétale contenant des acides gras poly-insaturés ou des acides gras poly-insaturés dérivés de manière enzymatique et d'une substance laitière contenant des protéines de lait par:
- une étape d'incorporation d'une huile végétale ou d'acides gras poly-insaturés dérivés de manière enzymatique avec une substance laitière pour obtenir un mélange d'une huile végétale ou d'acides gras poly-insaturés dérivés de manière enzymatique et d'une substance laitière,
- une étape d'homogénéisation du mélange ci-dessus mentionné pour obtenir un mélange homogénéisé d'une huile végétale ou d'acides gras poly-insaturés dérivés de manière enzymatique et d'une substance laitière,
- une étape de fermentation du mélange ci-dessus mentionné pour obtenir un mélange homogénéisé fermenté d'une huile végétale ou d'acides gras poly-insaturés dérivés de manière enzymatique et d'une substance laitière,

25. Composition qui peut être obtenue par le procédé selon la revendication 23 or 24.

26. Composition selon l'une des revendications 1 à 21 ou 25, comprenant de la vitamine E à une concentration de 1,5 à 600mg / 100g.
